Europäisches Patentamt

European Patent Office

Office européen des brevets

(11) Veröffentlichungsnummer: **0 281 656 B1**

(12) **EUROPÄISCHE PATENTSCHRIFT**

(45) Veröffentlichungstag der Patentschrift: **29.01.92**

(21) Anmeldenummer: **87103561.4**

(22) Anmeldetag: **12.03.87**

(51) Int. Cl.⁵: **A61K 35/78**

(54) Verwendung von Petasites-Extrakten zur Herstellung eines Arzneimittels zur Behandlung von gastrointestinalen Erkrankungen.

(43) Veröffentlichungstag der Anmeldung:
**14.09.88 Patentblatt 88/37**

(45) Bekanntmachung des Hinweises auf die
Patenterteilung:
**29.01.92 Patentblatt 92/05**

(84) Benannte Vertragsstaaten:
**AT BE CH DE ES FR GB GR IT LI LU NL SE**

(56) Entgegenhaltungen:
**CH-A- 228 340**
**DE-A- 1 911 862**
**DE-C- 897 145**
**FR-M- 8 351**

**CHEMICAL ABSTRACTS, Band 100, 1984, Seite 365, Zusammenfassung Nr. 197780s, Columbus Ohio, US; & RO-A-80 362 (INTREPRINDEREA DE MEDICAMENTE "BIOFARM") 30-01-1983**

(73) Patentinhaber: **Plantamed Arzneimittel GmbH**
**Kerschensteiner Strasse 11-15**
**W-8430 Neumarkt/Oberpfalz(DE)**

(72) Erfinder: **Brune, Kay, Prof.**
**Weiherackerweg 12**
**W-8525 Marloffstein(DE)**
Erfinder: **Peskar, Bernhard Alfred, Prof.**
**Herderallee 12**
**W-4630 Bochum 1(DE)**
Erfinder: **Vergin, Hartmut, Dr.**
**Ebenreuther Strasse 32**
**W-8500 Nürnberg(DE)**
Erfinder: **Ahrens, Kurt Henning, Dr.**
**Parterstrasse 9**
**W-8500 Nürnberg 80(DE)**
Erfinder: **Grätzel von Grätz, Jochen, Dr.**
**Ulmenstrasse 29**
**W-8501 Feucht(DE)**

(74) Vertreter: **Matschkur, Peter Dipl.-Phys. et al**
**Czowalla - Matschkur Patentanwälte Dr. Kurt-Schumacher-Strasse 23**
**W-8500 Nürnberg 11(DE)**

## Beschreibung

Die Erfindung betrifft die Verwendung von mittels lipophiler oder hydrophiler Extraktionsmittel gewonnener Petasites-Extrakte zur Herstellung eines Arzneimittels zur Behandlung von exogenen und endogenen Schädigungen der Magen-und Darmmukosa, gastrointestinalen Ulzerationen (Ulcusduodeni, Ulcus ventricoli) von Gastritiden jedweder Genese sowie Colitis ulcerosa und Morbus Crohn.

Die Pestwurz, Petasites hybridus oder Petasites officinalis (L.) Moendi (Astrales), wurde bereits in der Volksheilkunde wegen ihrer schleimlösenden Eigenschaften bei Husten und Asthma sowie wegen ihrer spasmolytischen Wirkung bei Krampfzuständen und Darmspasmen therapeutisch verwendet. Die spasmolytische Wirkung der Pflanze wurde in neuerer Zeit von Bucher (Arch. exper. Path. Pharmak. 213, 69 (1951), Crema et al. (Il Farmaco 12, 726 (1957) und Hörhammer et al. (zit. in Karl, J.: Naturheilpraxis 24, 290 (1971) experimentell nachgewiesen. Verwendung finden sowohl die Blätter als auch die Wurzeln und die ganze Pflanze. In der pharmazeutischen Stoffliste, 7. Auflage (1985; Werbeund Vertriebsgesellschaft Deutscher Apotheker mbH, 6 Frankfurt/Main 7) sind 15 Präparate enthalten, in denen Extrakte von Petasites hybridus allein oder in Kombination mit anderen Drogen arzneilich bei obengenannten Indikationen verwendet werden. Als erweiterte Anwendungsgebiete für Petasites hybridus gelten ferner Krankheitszustände, bei denen ein krampflösendes Schmerzmittel mit beruhigender und vegetativ regulierender Komponente angebracht ist wie: Vasomotorischer Spannungskopfschmerz, Migräne, Nacken- und Rückenschmerzen beim Zervikalsyndrom und bei Bandscheibenschäden, Koronarspasmen, pektanginöse Beschwerden, Altersherz, Lungenemphysen, Erkältungs- und Infektionskrankheiten. Nach Stoll et al. (Experientia 12, 360 (1956) und Aebi et al. (Pharm. Acta Helv. 30, 277 (1955), Pharm. Weekblad 93, 397 (1958) und: Über die Inhaltsstoffe von Petasites hybridus (2.) Fl. Wett., Verlag Haelbing und Lichtenhalm, Basel (1959) sind für die spasmolytische Wirkung vornehmlich die Angelikasäureester von Sesquiterpenalkoholen, Petasin und Isopetasin verantwortlich, wobei dem Petasin die wesentlich stärkere Wirksamkeit zukommt. Petasin und Isopetasin werden dabei gleichzeitig zur Standardisierung der Petasites-Extrakte durch Gaschromatographie (Wagner et al., Deutsche Apotheker-Zeitung 116 (28), 1009 (1976) herangezogen.

Der DE-C 897 145 ist ein Verfahren zur Herstellung eines Heilmittels aus Extrakten von Radix Petasitidis officinalis zu entnehmen. Gemäß der Lehre dieser Vorveröffentlichung wird, ausgehend von einem Pestwurzextrakt, welches sich bei Verwendung von Methanol als Extraktionsmittel ergibt, so viel Alkali zugegeben, daß der zunächst saure Auszug alkalisch reagiert. Darauf folgt eine Präzipitation mit Aceton und schließlich eine Trocknung des Präzipitats. Das erhaltene Produkt zeigt starke spasmolytische Wirksamkeit, weitere Eigenschaften des Wirkstoffs werden nicht genannt.

In Chemical Abstracts, Bd. 100, 1984, Nr. 197780s ist ein Verfahren zur Herstellung eines Arzneimittels aus den Wurzeln von Petasites hybridus beschrieben. Zum Zubereiten dieses Arzneimittels werden die Pflanzenteile zunächst getrocknet, pulverisiert und einer wiederholten Extraktion mit kaltem $Me_2CO$ unterzogen. Die Lösung wird gefiltert und durch Destillation konzentriert. Schließlich wird das Produkt granuliert und in Tablettenform gepreßt. Gemäß den Angaben in obiger Vorveröffentlichung entfaltet das Medikament spasmolytische Wirkung, weitere Effekte werden nicht erwähnt.

Ein anderes Verfahren zur Herstellung eines therapeutisch wirksamen Präparats aus Radix Petasitis officinalis offenbart die CH-A-228 340. Hierbei wird Radix Petasitis officinalis extrahiert und anschließend eine mehrstündige Behandlung mit ultravioletten Strahlen unter Zugabe von Sauerstoff durchgeführt. Ein nach diesem Verfahren hergestelltes Produkt wirkt auf Lymphdrüsen und -bahnen zusammenziehend. In der selben Patentschrift wird diese Wirkung als nützlich für die Therapie von Infektionskrankheiten bezeichnet. Außerdem wird unter Verweis auf nicht näher spezifizierte klinische Versuche eine heilende Wirkung bei Carzinom, Geschwulst und Geschwüren sowie verschiedenen Asthmaarten und eine Verschiebung der Eiweißstoffe im Blutplasma berichtet. Aufgrund des Anmeldedatums dieser Patentschrift von 1942 ergibt sich, daß ausschließlich Geschwüre an der Körperoberfläche gemeint sein können, da nur diese zum damaligen Zeitpunkt der Inspektion zugängig waren. Daß man die Angabe "Geschwüre" auf "Geschwüre an der Körperoberfläche" einschränken muß, folgt auch aus der zum Anmeldungszeitpunkt herrschenden Meinung, eine fehlende Lymphdränage sei ursächlich an der Entstehung dieser Geschwüre beteiligt. Darüber hinaus ist nach der CH-A-228 340 zur Herstellung des wirksamen Präparats eine Behandlung mit ultravioletten Strahlen erforderlich, wogegen das unbehandelte Extrakt als noch nicht wirksam beschrieben wird.

Schließlich ist aus der FR-M-8 351 ein Verfahren zur Herstellung eines Wirkstoffs aus Petasites officinalis Moench bekannt. Diese Petasitespflanzen werden durch mehrstündige Mazeration mit Wasser von etwa 65°C und einem pH-Wert von etwa 4,5 extrahiert. Eine anschließende Präzipitation mit Chloroform dient zur Isolation der aktiven Substanz. Dieses Verfahren liefert einen analgetischen Wirkstoff, welcher eine einschläfernde Nebenwirkung, jedoch keinen toxischen Effekt aufweist. Andere Wirkungen dieser Substanz sind in dieser Vorveröffentlichung nicht beschrieben.

Es wurde nun überraschenderweise gefunden, daß mittels lipophiler oder hydrophiler Extraktionsmittel gewonnene Petasites-Extrakte auch für andere Indikationen, nämlich zur Behandlung von exogenen und endogenen Schädigungen der Magen- und Darmmukosa, gastrointestinalen Ulzerationen (Ulcus duodeni, Ulcus ventricoli) von Gastritiden jedweder Genese sowie Colitis ulcerosa und Morbus Crohn, verwendet werden können. Offenbar ist die erfindungsgemäß erzielte ulcus-und zytoprotektive Wirksamkeit der Petasites-Extrakte auf einen funktionellen Leukotrien (LT) $C_4$-Antagonismus zurückzuführen. Da eine vermehrte Bildung von $LTC_4$ und/oder anderen 5-Lipoxygenase-Produkten des Arachidonsäurewechsels in der Pathophysiologie gastrointestinaler Erkrankungen eine zentrale Rolle spielt (M.A. Bray, 1986, Agents and Action 19, 87), können durch die erfindungsgemäße Anwendung von Petasites-Extrakten gastrointestinale Erkrankungen, insbesondere exogene und endogene Schädigungen der Magen-und Darmmukosa, gastrointestinale Ulzerationen (Ulcus duodeni, Ulcus ventriculi), Gastritiden jedweder Genese sowie Colitis ulcerosa und Morbus Crohn, günstig beeinflußt werden.

Die der vorliegenden Erfindung zugrundeliegende Erkenntnis der zusätzlichen pharmakologischen und therapeutisch nutzbaren Eigenschaft des Petasites-Extrakts - nämlich der zyto- und ulkusprotektiven Wirksamkeit - muß aufgrund der Kenntnis des oben angeführten Standes der Technik als ausgesprochen überraschend angesehen werden.

Für die erfindungsgemäß erfolgende Herstellung von zur Behandlung von gastrointestinalen Erkrankungen geeigneten Arzneimitteln sind grundsätzlich alle auf übliche Weise erhaltenen Extrakte der Stammpflanze Petasites hybridus (L.) geeignet. Insbesondere werden als Ausgangsmaterialien für die Herstellung der Extrakte getrocknete oberirdische und/oder unterirdische Pflanzenteile der Stammpflanze verwendet. Bevorzugt werden Blätter oder Rhizome eingesetzt, die vor der Extraktion in üblicher Weise geschnitten oder gemahlen werden.

Als Extrakte sind beispielsweise dünnflüssige Fluid-Extrakte, dickflüssige Spissum-Extrakte und pulverförmige Trockenextrakte geeignet.

Zur Herstellung der Extrakte werden die üblichen Auszugsmittel verwendet, und zwar inbesondere Wasser allein, oder übliche eher lipophile Lösungsmittel, wie Methanol, Ethanol, Aceton, Petrolether, ebenfalls allein oder im Gemisch mit Wasser. Zur erfindungsgemäß erfolgenden Herstellung der Arzneimittel werden wäßrige oder wäßrigethanolische Extrakte besonders bevorzugt.

Die Herstellung der erfindungsgemäß verwendeten Extrakte erfolgt in an sich bekannter Weise durch Mazeration oder Perkolation in handelsüblichen Extraktoren. Der Extraktion können Verfahrensschritte zur Konzentrierung, Keimreduktion und/oder Trocknung nachgeschaltet werden.

Die Herstellung der Extrakte erfolgt grundsätzlich nach den bekannten Verfahrensweisen, wie sie beispielsweise in Technologie pflanzl. Arneizubereitungen von P.H. List und P.C. Schmidt, WVG Stuttgart, 1984, beschrieben werden.

Die Herstellung der zur Behandlung der gastrointestinalen Erkrankungen vorgesehenen Arzneimittel erfolgt unter Verwendung der Petasites-Extrakte in an sich bekannter Weise. So können insbesondere Darreichungsformen, wie Tabletten, Kapseln, Granulate, Tropfen oder Säfte zur oralen Anwendung, Suppositorien zu rektalen und - nach angemessener Reinigung des Extrakts - Lösungen zur parenteralen Anwendung hergestellt werden.

Eine Dosiseinheit des erfindungsgemäß hergestellten Arzneimittels enthält im allgemeinen 25 bis 250 mg, vorzugsweise 50 bis 150 mg, Petasin-Extrakt. Die Dosiseinheit wird im allgemeinen drei- bis viermal täglich und über einen Zeitraum von beispielsweise 7 bis 14 Tagen erfolgen.

Im Einzelfall kann es aber gegebenenfalls erforderlich sein, von den vorgenannten Mengen abzuweichen, und zwar in Abhängigkeit vom individuellen Verhalten gegenüber dem Wirkstoff, Zustand des Patienten und dergleichen. So gibt es z.B. Fälle, wo mit weniger als der obengenannten Mindestmenge ausgekommen werden kann, während in anderen Fällen die genannte obere Grenze überschritten werden muß.

Die Herstellung der Arzneimittel erfolgt unter Verwendung üblicher Hilfsstoffe, wie Füll- und Trägerstoffe, Zerfallsförderer bzw. Sprengmittel, Gleit- und Schmiermittel, sowie geschmack- und farbgebender Komponenten für feste Zubereitungen sowie physiologisch verträglicher Lösungsmittel, Stabilisatoren, Konservierungsmittel oder Puffersubstanzen für flüssige Darreichungsformen.

Die Herstellung der Arzneimittel zur Behandlung der gastrointestinalen Erkrankungen erfolgt in üblichen Vorrichtungen und nach einschlägigen Verfahren.

Die ulkus- und zytoprotektiven Eigenschaften von Petasites-Extrakten wurden in folgenden in-vivo- und in-vitro-Modellen nachgewiesen.

Modell 1

Ethanol-induziertes gastromukosales Schädigungsmodell der Ratte

Männlichen Sprague-Dawley Ratten (SD-SIV, Fa. Savo, Kisslegg) der Gewichtsklasse 180-200 g, wurde für 16 Stunden das Futter (Altromin® Standarddiät für Ratten/Mäuse (Fa. Altromin, Lage/Lippe) entzogen, während Leitungswasser ad libitum zur Verfügung stand. Kontrollratten wurden mit 5 ml/kg 0.25 % Carboxymethylcellulose (CMC) und Testtiere mit unterschiedlichen Mengen von alkoholischen bzw. lyophilisierten und anschließend in 0.25 % CMC suspendierten Petasites-Extrakten i.g. behandelt. In einer ersten Untersuchungsreihe wurde ein handelsüblicher alkoholischer Extrakt geprüft, der 25 mg Extr. Petasites - auf 20 % Petas in eingestellt - pro ml enthielt.

Es wurden folgende Dosierungen geprüft: 0.033, 0.1 ml und 0.3 ml/ kg. Die Lösungen wurden in 0.25% CMC suspendiert und in einheitlichen Volumina von 5 ml/kg i.g. appliziert. Jeweils 30 Minuten nach i.g.-Gabe der Petasites-Extrakte bzw. der Vehikel-Suspension wurden 1.5 ml absolutes Ethanol oral instilliert. Nach 1 weiteren Stunde wurden die Tiere getötet, die Mägen entnommen, entlang der großen Kurvatur eröffnet und gespült. Das Ausmaß und die Beschaffeneit der Läsionen wurden nach einer von Marazzi-Uberti und Turba (C. Med. exp. 4, 284 (1961)) angegebenen Einteilung visuell ausgezählt und als Ulkus-Index ausgedrückt. Die Ergebnisse dieses Versuchs sind in der Tabelle 1 zusammengestellt.

| Dosierung (n= ) | Ulkus-Index (MW ± SEM) | % Hemmung | Signifikanz |
|---|---|---|---|
| 0.25 % CMC (7) | 45 ± 2.4 | - | |
| 0.3 ml/kg (6) (= 7.5 mg/kg) | 14 ± 2.8 | 69 | $p < 0.001$ |
| 0.1 ml/kg (7) (= 2.5 mg/kg) | 25 ± 3.9 | 44 | $p < 0.001$ |
| 0.033 ml/kg (6) (= 0.83 mg/kg) | 33 ± 1.5 | 27 | $p < 0.005$ |

Tabelle 1: Gastroprotektive Wirkung von Petasites-Extrakten am Ethanol-induzierten gastromukosalen Schädigungsmodell der Ratte.

Da im obigen Versuch der Petasites-Extrakt als ethanolische Lösung, in 0.25 % CMC suspendiert, verabreicht worden war, bestand die theoretische Möglichkeit einer adaptiven Zytoprotektion (D. Hollander, (1984) Forum DR. MED., Supplementum 12/84). Infolgedessen wurde das Lösungsmittel im Vakuum entfernt una der Petasites-Trockenextrakt in 0.25 % CMC suspendiert. Die sonstigen Versuchsbedingungen entsprachen den oben geschilderten. Geprüft wurde die Dosierung 15 mg/kg. Das Ergebnis ist in der

Tabelle 2 dargestellt.

| Dosierung (n= ) | Ulkus-Index (MW $\pm$ SEM) | % Hemmung | Signifikanz |
|---|---|---|---|
| 0.25 % CMC (7) | 47 $\pm$ 1.9 | - | |
| Extr. Petasitides 0.6 ml/kg (6) (= 15 mg/kg) | 13 $\pm$ 1.0 | 72 | p$<$ 0.001 |

**Tabelle 2:** Gastroprotektive Wirkung von Petasides-Trockenextrakt am Ethanol-induzierten gastromukosalen Schädigungs-modell der Ratte.

Der Vergleich der Ergebnisse der Tabellen 1 und 2 Zeigt eindeutig, daß der beobachtete zytoprotektive Effekt des Petasites-Extraktes unabhängig von seiner geprüften Form - d.h. ethanolische Lösung oder Trockenextrakt - ist. Anhand der Daten läßt sich eine approximative $ID_{50}$ von 3 mg/kg angeben. Die hohe gastroprotektive Wirksamkeit des Petasites-Extraktes am Ethanol-induzierten Schädigungsmodell wird besonders deutlich, wenn die an demselben Modell gefundenen $ID_{50\text{-Werte}}$ von Sucralfat = 102 mg/kg und dem antaziden $Al(OH)_{3\text{-Gel}}$ = 389 mg/kg aus der Literatur (I. Szelenyi et al. 1983, European J. Pharmacol. 88, 403) herangezogen werden.

Modell 2

Immobilisations-Stress induziertes gastromukosales Schädigungsmodell der Ratte

An diesem Modell wurden 2 unterschiedliche Petasites-Extrakte - I und II genannt - als ethanolische Lösungen in 1 % Tylose suspendiert, untersucht. Beide Extrakte waren auf ca. 20 % Petasin standardisiert. Der in hohen Dosen im Tierexperiment bekanntermaßen ulkus- bzw. zytoprotektiv wirksame $H_2$-Rezeptorantagonist Cimetidin (A. Robert et al. (1984) Scand. J. Gastroenterol. 19, (Suppl. 101), 69) wurde als Vergleichssubstanz mituntersucht.
Männlichen Sprague-Dawley Ratten (SD-SIV, Fa. Savo/Kisslegg) von einem durchschnittlichen Gewicht von 200-220 g wurde 16 h vor Versuchsbeginn das Futter bei freiem Zugang zu Leitungswasser entzogen. Gruppen von 6 Tieren erhielten im Abstand von 2 Stunden je 25, 12.5, 6.3 und 3.1 mg Petasites-Extrakt/kg Körpergewicht i.g. Kontrollratten erhielten zweimal 1 ml Tylose MH 300 (Methyl-hydroxyethyl-Zellulose) i.g. Cimetidin wurde ebenfalls im Abstand von 2 Stunden zu je 50 mg/kg i.g. dosiert. Gestreßt wurden die Tiere unmittelbar nach Applikation der zweiten Dosis durch Immobilisation in geeigneten Käfigen und Eintauchen derselben für 18 Stunden in Wasser von 23° C.
Nach Versuchsende wurden den Tieren die Mägen in Ethernarkose entnommen. Die Mägen wurden entlan der großen Kurvatur eröffnet und gespült. Ausmaß und Beschaffenheit der Läsionen wurde wie unter Modell 1 beschrieben visuell ausgewertet. Die Ergebnisse sind in der Tabelle 3 wiedergegeben.

| Substanz | Dosis mg/kg | Zahl der Tiere | Ulkus-Index (MW ± SD) | % Hemmung |
|---|---|---|---|---|
| Tylose | - | 6 | 83.2 ± 15.6 | - |
| Petasites-Extrakt I | 2 x 25 | 6 | 34.9 ± 9.1* | 58 |
|  | 2 x 12.5 | 6 | 44.9 ± 9.6* | 46 |
|  | 2 x 6.3 | 6 | 53.8 ± 3.7* | 35.4 |
|  | 2 x 3.1 | 6 | 58.8 ± 8.4* | 29.3 |
| Tylose | - | 6 | 66.8 ± 15.4 | - |
| Petasites-Extrakt II | 2 x 25 | 6 | 28.7 ± 10.1* | 57.1 |
|  | 2 x 12.5 | 6 | 39.0 ± 8.6* | 41.6 |
|  | 2 x 6.3 | 6 | 45.2 ± 8.1* | 32.4 |
|  | 2 x 3.1 | 6 | 51.2 ± 11.9 | 23.4 |
| Tylose | - | 9 | 72.9 ± 6.8 | - |
| Cimetidin | 2 x 50 | 9 | 35.0 ± 11.1* | 52 |

* signifikanter Unterschied zu Tylose-behandelten Kontrolltieren ($p < 0.05$).

Tabelle 3: Protektive Wirkung zweier Petasites-Extrakte (I und II) im Vergleich zu Cimetidin am Stress-induzierten gastro-mukosalen Schädigungsmodell der Ratte. (Dosierungsintervall: 2 h).

Wie die Ergebnisse zeigen, wirkt der Petasites-Extrakt auch an diesem Modell gastroprotektiv. Die approximative $ID_{50}$ läßt sich zu 2 x 13 mg/kg berechnen. Für Cimetidin ergibt sich eine ca. 50 %ige Hemmung von Immobilisations-Stress induzierten Läsionen bei der gewählten Dosierung von 2 x 50 mg/kg Körpergewicht. Demnach ist der Petasites-Extrakt in diesem Modell ca. 4 mal wirksamer als die Referenzsubstanz Cimetidin.

Modell 3

Indometacin-induziertes intestinales Schädigungsmodell bei der Ratte.

Gruppen von 5 männlichen, normal ernährten Sprague-Dawley Ratten (SD-SIV, Fa. Savo/Kisslegg) erhielten 8 mg/kg Indometacin zusammen mit den Testsubstanzen bzw. dem Vehikel 1 % Tylose MH 300 (Methyl-hydroxyl-Zellulose) intragastral appliziert. Nach 6 Stunden wurde erneut appliziert. Der Petasites-Extrakt wurde in folgenden Dosierungen geprüft: 25, 12.5 und 63 mg/kg Körpergewicht. Cimetidin wurde als Vergleichssubstanz in der Dosierung 2 x 50 mg/kg i.g. geprüft. 16 Stunden nach der letzten Substanzgabe wurden die Tiere mit Ether narkotisiert. Mit Ausnahme von Caecum und Colon wurde der gesamte Darm entfernt, sektionsweise longitudinal eröffnet, mit isotoner Kochsalzlösung gespült, abgetupft und gewogen. Als Parameter für die intestinale Schädigung durch Indometacin wurde das Gewichtsverhältnis aus geschädigten und intakten Darmanteilen gewählt. Die Ergebnisse sind in der nachfolgenden Tabelle 4 wiedergegeben.

| Substanz | Dosis mg/kg | Zahl der Tiere | % Ulzeration pro gesamten Dünndarm | % Hemmung |
|---|---|---|---|---|
| Tylose | - | 5 | $80.1 \pm 7.7$ | - |
| Petasites-Extrakt I | 2 x 25 | 5 | $18.1 \pm 6.6*$ | 77.4 |
| | 2 x 12.5 | 5 | $46.1 \pm 10.1*$ | 42.4 |
| | 2 x 6.3 | 5 | $53.4 \pm 7.4*$ | 33.3 |
| Tylose | - | 5 | $73.1 \pm 10$ | - |
| Cimetidin | 2 x 50 | 5 | $39.4 \pm 11.9*$ | 54 |

* signifikanter Unterschied zu Tylose-behandelten Kontrolltieren ($p < 0.05$).

Tabelle 4: Protektive Wirkung des Petasites-Extraktes gegenüber Indometacin-indzierten intestinalen Läsionen bei der Ratte.

Wie die Ergebnisse zeigen, vermag der Petasites Extrakt die durch zweimalige Gabe von Indometacin verursachten intestinalen Läsionen bei der Ratte deutlich zu hemmen. Die approximative $ID_{50}$ (50 %-Hemmdosis) läßt sich in 2 x 14.5 mg/kg berechnen. Vergleicht man die Wirksamkeit des Petasites-Extraktes in diesem Modell mit derjenigen von Cimetidin ($ID_{50}$ = 2 x 208 umol/kg entsprechend ca. 2 x 50 mg/kg), so ergibt sich eine 3fach stärkere Wirksamkeit des Petasites-Extraktes auf Gewichtsbasis.

Modell 4

Freisetzung von immunoreaktivem Prostaglandin (PG)E$_2$
und Leukotrien (LT) C$_4$ aus Makrophagen in vitro

Unstimulierte Makrophagen wurden aus dem Bauchraum von männlichen NMRI-Mäusen, die ein durchschnittliches Körpergewicht von 20 g aufwiesen, gesammelt. 2 x 10$^6$ Zellen wurden in 35 mm Petrischalen in 2 ml nach Dulbecco modifiziertem Eagle-Medium (DMEM), welches 10 % hitzeinaktiviertes fetales Kälberserum enthielt, bei 37˚C in mit 5 % CO$_2$ angereicherter Luft inkubiert. Danach wurden nicht-adhärente Zellen durch Waschen entfernt und 2 ml frisches DMEM-Medium (ohne Kälberserum) hinzugefügt. Dieses Medium enthielt die verwendeten Lösungsmittel (Ethanol + Dimthylsulfoxid (DMSO), oder nur DMSO).

PGE$_2$ and LTC$_4$ wurden durch Ca-Ionophor (A 23187) als Stimulus freigesetzt. Zu diesem Zwecke wurde A 23187 in 10$^{-6}$ molarer Lösung dem DMEM-Medium hinzugefügt.

Der Petasites-Extrakt wurde in 50 oder 10 ul-Volumina (entsprechend 1.25 oder 0.25 mg) sowie in den Tabellen 5 und 6 angegebenen Verdünnungen dem DMEM-Medium hinzugesetzt. Nach 2 h Inkubationszeit wurden die Überstände aller Schalen gesammelt und in Glasfläschchen schockgefroren. Die verbleibenden Zellrasen wurden hinsichtlich ihrer Vitalität durch Trypanblau-Ausschluß überprüft.

Die tiefgefrorenen Überstände der kultivierten Makrophagen wurden auf ihren Gehalt hin an immunoreakti-ven PGE$_2$ und LTC$_4$ mit Hilfe spezifischer Radioimmunoassays (U. Aehringhaus et al. (1982) FEBS-LETT. 146, 111. K. Brune et al. (1981) Naunyn-Schmiedebergs Arch. Pharmacol. 315, 269) überprüft.

Die Ergebnisse sind in Form der Tabellen 5 und 6 zusammengefaßt.

| Substanz | Appl. Volumen pro Inkubations-ansatz (ul) | ng/ml im Kulturüberstand | |
|---|---|---|---|
| | | PGE | LTC |
| EtOH 70% + DMSO | 10 | $3.3 \pm 0.87$ | $< 1.44$ |
| EtOH 70% + A 23187 ($10^{-6}$ M) | 10 | $18.43 \pm 2.8$ | $38.64 \pm 1.10$ |
| Petasites-Extrakt (ethanolische Lösung) + A 23187 ($10^{-6}$ M) | 10 | $37.86 \pm 5.6$ o | $< 1.44$ x |
| | 1 | $24.28 \pm 3.0$ lo | $31.31 \pm 1.85$ x |
| | 0.1 | $13.26 \pm 2.23$ | $21.55 \pm 0.79$ x |
| | 0.01 | $15.44 \pm 1.83$ | $29.81 \pm 1.04$ x |
| | 0.001 | $14.65 \pm 2.25$ | $41.75 \pm 2.88$ |

x signifikante Hemmung der Bildung von $LTC_4$ ($p < 0.05$).

o signifikante Steigerung der Bildung von $PGE_2$ ($p < 0.05$).

1 ul ethanolische Lösung = 25 ug Petasites Extrakt. EtOH = Ethanol, DMSO = Dimethylsulfoxid.

Tabelle 5: Freisetzung von $PGE_2$ und $LTC_4$ aus Mäuseperitonealmakrophagen in den Kulturüberstand (MW $\pm$ SD; n = 5 pro Ansatz).

9

| | Appl. Volumen pro Inkubations-ansatz (ul) | ng/ml im Kulturüberstand | |
|---|---|---|---|
| Substanz | | PGE | LTC |
| DMSO | 50 | 0.23 | $<1.44$ |
| EtOH 70% + DMSO | 50 | 0.11 | $<1.44$ |
| DMSO + A 23 187 (10-6 M) | 50 | $17.29 \pm 1.06$ | $73.68 \pm 11.31$ |
| Petasites-Extrakt* + A 23187 (10-6 M) | 50 | $24.61 \pm 1.460$ | $<1.44$ x |
| | 20 | $16.36 \pm 2.37$ | $<1.44$ x |
| | 10 | $14.53 \pm 0.83$ | $<1.44$ x |
| | 1 | $18.43 \pm 4.31$ | $14.79 \pm 0.78$x |
| | 0.1 | $17.57 \pm 0.74$ | $22.83 \pm 4.32$x |

\* ethanolische Lösung zur Trockne gebracht, in DMSO aufgenommen. (1 ul = 25 ug Petasites-Extrakt).

x signifikante Hemmung der Bildung von $LTC_4$ ($p< 0.05$).

o signifikante Steigerung der Bildung von $PGE_2$ ($p<0.05$).

Tabelle 6: Freisetzung von $PGE_2$ und $LTC_4$ aus Mäuseperitonealmakro-phagen in den Kulturüberstand (MW $\pm$ SD; n = 5 pro Ansatz).

Nach dem Stand wissenschaftlicher Erkenntnis wirken zyto- bzw. gastroprotektive Substanzen zum einen über eine Stimulation der Prostaglandin (PG) - besonders der $PGE_2$-Synthese in der Magenmukosa (Robert, (1981), Prostaglandins Suppl. 21, 89 ).

Der Wirkmechanismus der durch Prostaglandine vermittelten Zytoprotektion ist noch weitgehend ungeklärt. Es kann jedoch angenommen werden, daß die Prostaglandine unter anderem über die Verstärkung der präepithelialen extrinsischen Faktoren wie der Beeinflussung der Mukus- und Bikarbonationensekretion wirken. Die Eigenschaft der Prostaglandine, die gastrale Bikarbonationensekretion zu fördern, wurde bereits von Smeaton et al. ((1982), Amer. J. Physiol. 245, G 751) beschrieben.

Neuerdings wird die Hemmbarkeit der gastralen und intestinalen Leukotrien (LT) $C_4$ Synthese als zusätzlicher bzw. als dominierender Wirkungsmechanismus diskutiert (Hawkey et al. (1985), GUT 26, A 1148. B.M. Peskar et al. (1986), 4. Prostaglandins 31 (2), 283), da vermehrt gebildetes $LTC_4$ ein wichtiger Mediator beispielsweise der Ethanol bedingten Schädigung der Magenscheimhaut zu sein scheint.

Die vorliegenden Ergebnisse zeigen, daß Petasites-Extrakte in höheren, therapierelevanten Dosen sowohl $PGE_2$ vemehrt aus mit Ca-Ionophor stimulierten Makrophagenkulturen freisetzen als auch die stimulierte $LTC_4$ Liberation deutlich hemmen. Bei 0.25 mg Petasites-Extrakt im Inkubationsmedium kommt die durch Ca-Ionophor stimulierte $LTC_4$ Freisetzung bereits vollkommen zum Erliegen.

Den in den Tabellen 5 und 6 dargestellten Ergebnissen ist weiterhin zu entnehmen, daß die Art der verwendeten Lösungsmittel (DMSO, 70 % Ethanol) keine Rolle spielt.

Die Erfindung wird in den Beispielen erläutert.

Herstellungsbeispiel für einen erfindungsgemäß verwendbaren Petasites-Extrakt

500 kg Petasites-Rhizom, geschnitten, werden in V2A Stahlkolonnen so lange mit Wasser von 60°C extrahiert, bis das abfließende Perkolat nur noch schwach gefärbt ist.

Die wäßrige Lösung wird am Dünnschicht-Plattenverdampfer konzentriert, in einer Kurzzeit-Pasteurisationsanlage pasteurisiert und das Konzentrat sprühgetrocknet.

**Beispiel 1**

Herstellung von Tabletten

| 1 Tablette enthält: | |
| --- | --- |
| Petasites-Extrakt, fein gepulvert | 150,0 mg |
| Mikrokristalline Cellulose | 127,5 mg |
| Hochdisperse Kieselsäure | 3,0 mg |
| Natriumcarboxymethylstärke | 15,0 mg |
| Magnesiumstearat | 4,5 mg |
| | 300,0 mg |

Die aufgeführten Substanzen werden in einem geeigneten Mischer gemischt und die Mischung auf einer Rundläufer-Tablettiermaschine zu Tabletten mit 10 mm Durchmesser verpreßt.

Herstellung von Tropflösungen

| 1 ml enthält: | |
| --- | --- |
| Petasites-Extrakt, fein gepulvert | 100,0 mg |
| Citronensäure | 20,0 mg |
| p-Hydroxybenzoesäurepropylester | 0,6 mg |
| Benzoesäure | 0,9 mg |
| Ethanol | 350,0 mg |
| Gereinigtes Wasser | 488,5 mg |
| | 960,0 mg |

Extrakt und pHB-Ester werden in Ethanol gelöst. Anschließend wird die Lösung der beiden Säuren in Wasser hinzugegeben und das Ganze vermischt.

**Patentansprüche**

1.  Verwendung von mittels lipophiler oder hydrophiler Extraktionsmittel gewonnener Petasites-Extrakte zur Herstellung eines Arzneimittels zur Behandlung von exogenen und endogenen Schädigungen der Magen- und Darmmukosa, gastrointestinalen Ulzerationen (Ulcusdodeni, Ulcusventricoli) Gastritiden jedweder Genese sowie Colitis ulcerosa und Morbus Crohn.

**Claims**

1.  Use of petasite extracts obtained by means of lipophilic or hydrophilic extraction agents for the manufacture of a medicine for treating exogenous and endogenous lesions of the gastric and intestinal mucosa, gastrointestinal ulcerations (duodenal ulcer, stomach ulcer), forms of gastritis of any origin, and colitis ulcerosa and Crohn's disease.

**Revendications**

1.  Utilisation d'extraits de petasites obtenus à l'aide d'agents d'extraction lipophiles ou hydrophiles pour la préparation d'un médicament destiné au traitement des lésions exogènes et endogènes de la muqueuse gastrique et intestinale, d'ulcérations gastro-intestinales (ulcère duodénal, ulcus-ventriculi), de gastrites de toutes origines ainsi que de la colite ulcéreuse et de la maladie de Crohn.